# EUROPEAN PATENT APPLICATION

(11) **EP 4 545 628 A1**
(43) Date of publication of application: **30.04.2025**
(21) Application number: 23861710.4
(22) Date of filing: 29.12.2023
(51) Int. Cl.: C12N 1/20, A61K 35/744, A61P 1/00, A23L 33/135, C12R 1/01

(54) **BREAST MILK-DERIVED LIMOSILACTOBACILLUS FERMENTUM MSJK0025 CAPABLE OF IMPROVING INTESTINAL FLORA CONDITION AND USE THEREOF**

(30) Priority: 23.08.2023 CN 202311070808
(71) Applicant: Zhejiang Minsheng Healthcare Co., Ltd., Huzhou, Zhejiang 313300 (CN); Hangzhou Minsheng Healthcare Co., Ltd., Hangzhou, Zhejiang 311100 (CN)
(72) Inventor: YANG, Shanyan, Hangzhou Zhejiang 311100 (CN); FENG, Fengjiang, Hangzhou Zhejiang 311100 (CN); LI, Xuelong, Hangzhou Zhejiang 311100 (CN); ZHU, Yuqi, Hangzhou Zhejiang 311100 (CN); ZHANG, Haijun, Hangzhou Zhejiang 311100 (CN)
(74) Representative: Ullrich & Naumann PartG mbB
(86) International application number: PCT/CN2023/143049
(87) International publication number: WO 2025/039454

(57) **Abstract**

The present application relates to a *Limosilactobacillus fermentum* MSJK0025 from breast milk for adjusting gut microbiota and an application thereof. The *Limosilactobacillus fermentum* MSJK0025 from breast milk is preserved in China General Microbiological Culture Collection Center, with a preservation number of CGMCC No.26426. The *Limosilactobacillus fermentum* from breast milk provided in the present application is a new strain separated from the healthy nursing woman, named as *"Limosilactobacillus fermentum* MSJK0025", having good antibiotics sensitivity, and its property can be stably inherited. It has good resistance to acid, bile salt, and artificial gastric juice and intestinal juice, and fast growth rate and good safety. At the same time, the strain has strong survival capability in the gut, and effectively inhibits pathogenic bacteria in the gut such as Escherichia coli, Staphylococcus aureus, Salmonella typhimurium, and Candida albicans, but does not inhibit the probiotic in the gut such as lactobacillus and bifidobacterium, so the strain has broad application prospect in adjusting the balance of gut microbiota.

## Description

### TECHNICAL FIELD

The present application relates to the technical field of microorganism, and in particular, relates to a *Limosilactobacillus fermentum* MSJK0025 from breast milk for adjusting gut microbiota and an application thereof.

### BACKGROUND ART

More and more researches indicate that symbiotic microbiota is closely related to the health and disease of parasitifer. The gut microbiota can be considered as "microbial organ" of body, which participates in multifaceted local and systemic physiological processes of parasitifer such as metabolism, immunity, endocrine, nerve by mechanisms such as microbiota composition, metabolites and secondary metabolites and symbiotic bacterial translocation, thereby having risks of diseases such as obesity, type II diabetes, fatty liver, cardiovascular disease, autoimmune and inflammatory diseases, mental and neurological disorders and cancer.

The breast milk, as an indispensable nutrient source for infants during the early stages of growth, plays a crucial role in growth and development of the infants. The presence of live bacteria in the breast milk has been confirmed. The microorganisms with probiotic properties in the breast milk are the basis for the infants to initially build an immune system and resist diseases, which replace the pathogenic bacteria in guts of the infants by competition exclusion and survive in guts, and improve intestinal environments of the infants due to their probiotic properties.

*Limosilactobacillus fermentum* is one of microorganisms from the breast milk, which belongs to Lactic acid bacteria, and is widely used in food, medicine, agriculture and other fields. For example, a famous strain *Limosilactobacillus fermentum* CECT5716 from Biosearch Life of Spain is a probiotic separated from the breast milk, which can prevent and treat mastitis during the lactation period, alleviate swelling and pain of the breasts during the lactation period, improve immunity and gut health of mother and baby. The strain has been listed in the list of biological agents recommended by EU Qualified Presumption of Safety (QPS), and listed in the "list of microorganisms with a history of safe use in food" of International Dairy Federation (IDF). In 2011, *Limosilactobacillus fermentum* has been listed in Chinese "List of strains for food". Moreover, the strains have been identified by Generally Recognized as Safe (GRAS) of Food and Drug Administration, which can be used in the infant formula. The infant formula containing Lactobacillus fermentum CECT5716 are sold at many countries (regions) including Europe and Asia.

However, the health benefits of probiotics have strain diversity, that is, strains belonging to the same species may generate different effects on human body. It has been confirmed in some researches that some strains of Lactobacillus fermentum may have negative impact on human body, for example, the integrity of gut cell barrier may be decreased. In addition, the probiotics are generally administrated orally, if their probiotic properties need to be developed, they must pass the ordeal of gastrointestinal barrier (especially stomach acid and bile). The bile is secreted by the liver and enters "the duodenum (a part of the small gut)" through the gallbladder, which has good killing effect to the probiotics. A concentration of the bile salt in the duodenum fluctuates is between 0.03%-0.3%. However, a retention time of the food in the duodenum is about 4-6 h. In normal circumstance, the pH of gastric juice during fasting is 0.9-1.5, and the pH of gastric juice after a meal is above 3.5. However, the gastric emptying time is about 3 h. Some current probiotic strains, including Lactobacillus fermentum have different stress resistance, which directly affects the efficacy of probiotics. At the same time, drug-resistance to antibiotic is one important index of safety evaluation of the probiotics. Since current probiotic strains have different separation sources, and their genomes commonly contain drug-resistance gene, they have drug-resistance to antibiotic and further have potential safety hazard. Even current genomes of the strains contain some virulence factors, although they are safety after oral acute toxicity test, there is still a possibility of "gene migration". Therefore, there is a potential risk. The probiotic need to survive in the gut for function, but the main influence factors for survival of the probiotic in the gut are the resistance to gastric juice and bile salt. Some current probiotic strains have different resistance, so that they are difficult to survive in the gut. The above disadvantages of the current probiotics greatly affect and restrict the probiotics to play their probiotic properties in actual application.

### SUMMARY

In order to overcome the disadvantages of the prior art, the present application provides *a Limosilactobacillus fermentum* MSJK 0025 from breast milk for adjusting gut microbiota. The strains were separated from the breast milk of the healthy nursing woman at Linping District, Hangzhou City, Zhejiang Province in September 2021, which have good resistance to acid, bile salt, artificial gastric juice and intestinal juice and strong survival capability in the gut, so that they can effectively inhibit Escherichia coli, Staphylococcus aureus, Salmonella typhimurium, Candida albicans and other pathogenic bacteria in the gut, but have no inhibition effect to lactobacillus and bifidobacterium, thereby having broad application prospect in adjusting gut microbiota.

For this, in a first aspect, the present application provides a *Limosilactobacillus fermentum* MSJK0025 from breast milk for adjusting gut microbiota, and the *Limosilactobacillus fermentum* MSJK0025 is preserved in China General Microbiological Culture Collection Center, with a preservation number of CGMCC No.26426.

The strains in the breast milk of the healthy nursing woman are separated and screened to obtain a non-hemolytic strain with good acid resistance, artificial gastric juice resistance, bile salt resistance, and artificial intestinal juice resistance. From the test data such as cell morphology, physiological and biochemical characteristics, 16 S rRNA gene sequence (a sequence number in GenBank is OP861710.1), by reference to some related research papers such as "Bergry's Manual of Systematic Bacteriology" and "International Journal of Systematic and Evolutionary Microbiology", this strain is identified as *"Limosilactobacillus fermentum"* and officially named as *"Limosilactobacillus fermentum* MSJK0025". The strain is preserved in China General Microbiological Culture Collection Center, with a preservation number of CGMCC No.26426.

In some embodiments, 16S rRNA nucleotide sequence of the *Limosilactobacillus fermentum* MSJK0025 from breast milk is shown in SEQ ID NO:1, with a login number of OP861710.1 in GeneBank.

In the present application, 16S rRNA nucleotide sequence is 16S rRNA gene sequence of the *Limosilactobacillus fermentum* MSJK0025 from breast milk.

In some embodiments, a login number of a complete genome sequence of the *Limosilactobacillus fermentum* MSJK0025 from breast milk is CP121468.1 in GeneBank.

In the present application, after analysing the data of the complete genome sequence of the *Limosilactobacillus fermentum* MSJK0025 from breast milk, the results show that: the virulence factors are not detected, so the strain has no pathogenic hazards; the antibiotic resistance genes are not detected, so the strain has good antibiotics sensitivity and its characteristics can be stably inherited; and the strain can generate exopolysaccharide which facilitates survival in gut and have strong survival capability in the gut, and these are the foundation for playing probiotic property. The discovery of this strain facilitates breaking the monopoly to Chinese probiotic market from European, American and other countries.

In a second aspect, the present application provides an application of the *Limosilactobacillus fermentum* MSJK0025 from breast milk disclosed in the first aspect of the present application in preparing probiotic composition for inhibiting pathogenic bacteria in the gut.

In the present application, the *Limosilactobacillus fermentum* MSJK0025 from breast milk has strong survival capability in the gut and obvious inhibition effect to the pathogenic bacteria in the gut, which can be better used for preparing the probiotic composition which adjusts gut microbiota.

In some embodiments, the pathogenic bacteria in the gut includes one or more selected from the group consisting of Escherichia coli, Staphylococcus aureus, Salmonella typhimurium, and Candida albicans.

It can be seen from the inhibition zone test that, the *Limosilactobacillus fermentum* MSJK 0025 from breast milk has an inhibition effect to the pathogenic bacteria in the gut such as Escherichia coli, Staphylococcus aureus, Salmonella typhimurium, and Candida albicans, and their inhibition effects is that: Escherichia coli > Staphylococcus aureus > Salmonella typhimurium > Candida albicans. However, the *Limosilactobacillus fermentum* MSJK 0025 from breast milk has no inhibition effect to the probiotic in the gut such as lactobacillus and bifidobacterium.

In some embodiments, the probiotic composition is an oral formulation.

In the present application, the *Limosilactobacillus fermentum* MSJK 0025 from breast milk has good acid resistance, artificial gastric juice resistance, artificial intestinal juice resistance, and bile salt resistance, so the probiotic composition containing the *Limosilactobacillus fermentum* MSJK 0025 from breast milk can be an oral formulation. The *Limosilactobacillus fermentum* MSJK 0025 can play probiotic property by oral formulation.

In a third aspect, the present application provides a probiotic composition containing the *Limosilactobacillus fermentum* MSJK 0025 from breast milk disclosed in the first aspect of the present application.

In the present application, the *Limosilactobacillus fermentum* MSJK 0025 from breast milk can generate exopolysaccharide which facilitates survival in gut and have strong survival capability in the gut, and this is the foundation for playing a role in adjusting the gut microbiota. In addition, in order to play a probiotic property, the probiotic needs to arrive at small intestine and large intestine as survival status to deal with multiple stressors in the digestive tract. The *Limosilactobacillus fermentum* MSJK 0025 from breast milk in the present application has strong acid resistance and bile resistance, which can effectively deal with the acid environment in stomach and bile. Therefore, the *Limosilactobacillus fermentum* MSJK 0025 from breast milk in the present application can survive in the gut due to its adhesion, has an interaction relationship with the parasitifer, and play its probiotic property, so it can be as an effective probiotic composition.

In a fourth aspect, the present application provides a pharmaceutical composition for adjusting balance of gut microbiota, which includes the *Limosilactobacillus fermentum* MSJK0025 from breast milk disclosed in the first aspect of the present application.

In the present application, the *Limosilactobacillus fermentum* MSJK0025 from breast milk can effectively survive in the gut, which has obvious inhibition effect to the pathogenic bacteria in the gut (for example, Escherichia coli, Staphylococcus aureus, Salmonella typhimurium and Candida albicans), but has no inhibition effect to the beneficial bacteria (for example, Lactobacillus plantarum, Bifidobacterium longum and Lactobacillus acidophilus), so the pharmaceutical composition containing the *Limosilactobacillus fermentum* MSJK0025 from breast milk has better effect for adjusting balance of gut microbiota.

In the present application, the pharmaceutical composition further includes a pharmaceutically acceptable carrier. In the present application, the pharmaceutically acceptable carrier includes but is not limited to excipients, surfactants, desiccants, diluents, supporting agents, adjuvants or additives which are accepted by pharmacy. The suitable pharmaceutically acceptable carrier can be, for example, magnesium carbonate, lactose, pectin, dextrin, starch, astragalus gum, methyl cellulose, hydroxypropyl methyl cellulose, carboxymethyl cellulose sodium, and cocoa butter. In the present application, the pharmaceutically acceptable carrier can be solid, semi-solid or liquid.

In a fifth aspect, the present application provides a functional food for adjusting the balance of gut microbiota, which includes the *Limosilactobacillus fermentum* MSJK0025 from breast milk disclosed in the first aspect of the present application.

As mentioned above, the *Limosilactobacillus fermentum* MSJK0025 from breast milk disclosed in the present application can effectively survive in the gut, and has obvious inhibition effect to pathogenic bacteria in the gut and has no inhibition effect to the beneficial bacteria in the gut. Therefore, the *Limosilactobacillus fermentum* MSJK0025 from breast milk disclosed in the present application is further applied in the functional food for adjusting the balance of gut microbiota.

In the present application, the functional food can be, for example, fermented milk, fermented fruits and vegetables, or ferment.

The beneficial technical effects of the present application are that: the *Limosilactobacillus fermentum* from breast milk provided in the present application is a new strain separated from the healthy nursing woman, named as *"Limosilactobacillus fermentum* MSJK0025", having good antibiotics sensitivity, and its property can be stably inherited. It has good resistance to acid, bile salt, and artificial gastric juice and intestinal juice, and fast growth rate and good safety (no hemolysis, no virulence factor detected and no antibiotic resistance genes detected). At the same time, the strain can generate exopolysaccharide which facilitates survival in gut and have strong survival capability in the gut, and effectively inhibit pathogenic bacteria in the gut and have the effect to adjust the balance of gut microbiota and broad application prospect. In addition, the *Limosilactobacillus fermentum* MSJK0025 from breast milk disclosed in the present application has good probiotic property, which can enhance the development of localized probiotic strains and facilitate breaking the monopoly to Chinese probiotic market from European, American and other countries.

### BRIEF DESCRIPTION OF THE DRAWINGS

FIG. 1 is a plate colony picture after culturing the breast milk with different dilution gradient according to Example 1.
FIG. 2 is a picture after treating the strain MSJK0025 of Example 1 by streak plate method.
FIG. 3 is a picture of bacterial colony of the strain MSJK0025 of Example 1 on a plate.
FIG. 4 is a microscopic examination picture of the strain MSJK0025 of Example 1 after Gram staining.
FIG. 5 is a scanning electron microscopy picture of the strain MSJK0025 of Example 1.
FIG. 6 is a plate picture of hemolysis-detection of *Limosilactobacillus fermentum* MSJK0025 of Example 2.
FIG. 7 is a typical image of the fluorescence intensity of zebrafish gut treated by sample *(Limosilactobacillus fermentum* MSJK0025) of Example 6.
FIG. 8 is a fluorescence intensity of zebrafish gut treated by sample (*Limosilactobacillus fermentum* MSJK0025) of Example 6 at different time.
FIG. 9 is an antibacterial effect picture of *Limosilactobacillus fermentum* MSJK0025 of Example 7 to Escherichia coli, Staphylococcus aureus, Salmonella typhimurium, Candida albicans, Lactobacillus plantarum, Bifidobacterium longum and Lactobacillus acidophilus.

Biological preservation information
*Limosilactobacillus fermentum* MSJK0025 has a preservation number of CGMCC No.26426, the preservation unit is the China General Microbiological Culture Collection Center (referred to as CGMCC, the address is No. 3, Courtyard 1, Beichen West Road, Chaoyang District, Beijing), and the preservation time is January 9, 2023.

### DETAILED DESCRIPTION

In order to understand easily, the present application will be further described in detail below in combination with the examples, and these examples are only used for illustration, but not limit to the application scope of the present application. All the raw materials used in the present application are commercially available or prepared by conventional method if no special illustration.

### Example 1: the separation, screening and identification of the Limosilactobacillus fermentum MSJK0025

### 1. Separation and purification

Since most of the current probiotics are lactic acid bacteria, the lactic acid bacteria, as the target strain, are separated and screened. The lactic acid bacteria will generate lactic acid and other organic acid during the growth metabolism. If calcium carbonate is added in the MRS culture medium, clear zones - "calcium dissolving zone" are formed around bacterial colony when they are cultured at the plate, which can be an indicator for initial screening of lactic acid bacteria. The larger calcium dissolving zone, the stronger the acid production ability. Therefore, the bacterial colony with a larger calcium dissolving zone are selected to be streaked and separated. The specific separation and purification method is as follows.

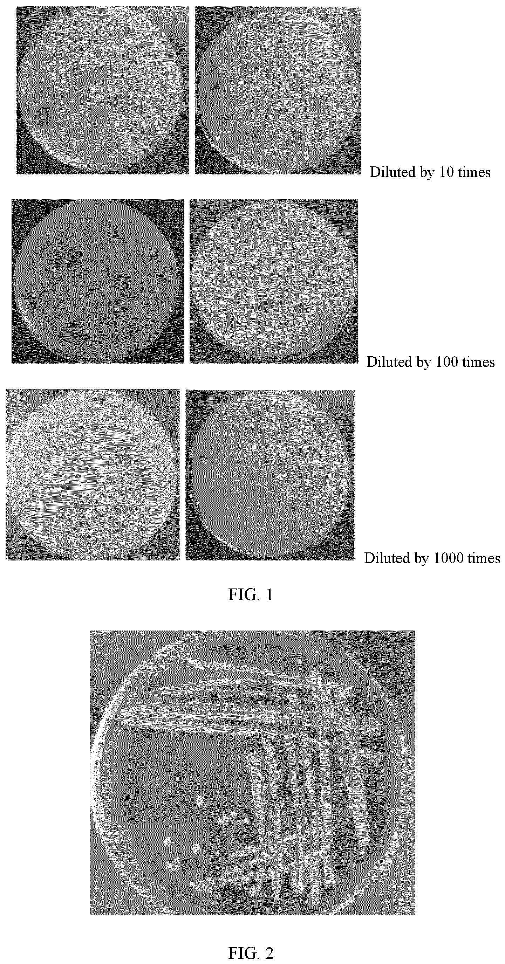

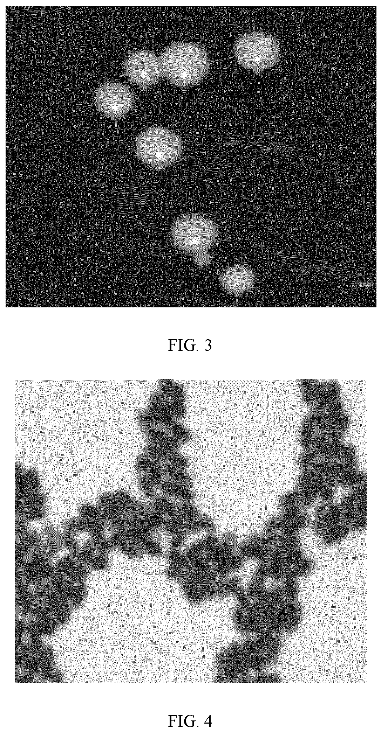

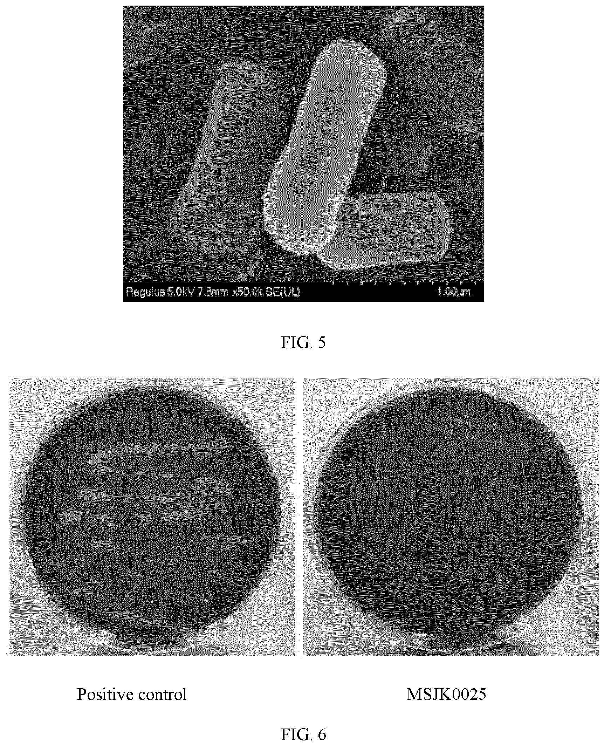

The fresh breast milk sample was diluted in gradient by sterile saline to 10⁻¹,10⁻² and 10⁻³, and coated on the MRS culture medium containing 0.3% CaCO₃. Two samples with the same gradient were as parallel sample. The coated plates were cultured in a bacterial incubator of 36 °C for 48 h, and the bacterial colony cultured on plate with different gradients were shown in FIG.1. The white or milk white, and well growing single strain with obvious clear zones - "calcium dissolving zone" were selected to be conducted Gram staining and microscopic examination, and the rod-shaped strains with positive Gram staining were selected, streaked and separated for more than 5 times to obtain the purified strains. In particular, the streaked and separated picture of strain MSJK0025 was shown in FIG.2, and the picture of bacterial colony of the strain MSJK0025 was shown in FIG.3. The microscopic examination picture of the strain MSJK0025 of Example 1 after Gram staining was shown in FIG.4, and the scanning electron microscopy picture of the strain MSJK0025 was shown in FIG.5. As can be seen from FIGS. 4-5 that, the microscopic examination picture of Gram staining showed that the strain MSJK0025 was Gram positive strain and rod-shaped, while the scanning electron microscopy picture shown that the strain MSJK0025 was rod-shaped, which had smooth surface, no cilia or flagella and no spores. Finally, the purified strains obtained after separation were conducted enrichment culture in the bacterial incubator of 36 °C, and bacterial fluid and sterile glycerol aqueous solution with a concentration of 50% were mixed by a ratio of 1:1, and persevered in an ultra-low temperature freezer of -86 °C.

### 2. Sequencing and initial screening of 16S rRNA

All the separated single strains were conducted 16S rRNA sequencing, after comparison, it was found that the 16S rRNA gene sequence of the MSJK0025 strain had high similarity with that of *Limosilactobacillus fermentum,* which reached up to 100% (the compared strain was *Limosilactobacillus fermentum* (CIP 102980)). Therefore, it was preliminarily identified as *Limosilactobacillus fermentum* with a number of MSJK0025. In particular, the 16S rRNA gene sequence of the MSJK0025 strain was shown as follows:

AATTGATTGATGGTGCTTGCACCTGATTGATTTTGGTCGCCAACGAGTGGCGGACGG

GTGAGTAACACGTAGGTAACCTGCCCAGAAGCGGGGGACAACATTTGGAAACAGAT

GCTAATACCGCATAACAACGTTGTTCGCATGAACAACGCTTAAAAGATGGCATATCGC

TATCACTTCTGGATGGACCTGCGGTGCATTAGCTTGTTGGTGGGGTAACGGCCTACCA

AGGCGATGATGCATAGCCGAGTTGAGAGACTGATCGGCCACAATGGGACTGAGACAC

GGCCCATACTCCTACGGGAGGCAGCAGTAGGGAATCTTCCACAATGGGCGCAAGCCT

GATGGAGCAACACCGCGTGAGTGAAGAAGGGTTTCGGCTCGTAAAGCTCTGTTGTA

AAAGAAGAACACGTATGAGAGTAACTGTTCATACGTTGACGGTATTTAACCAGAAAG

TCACGGCTAACTACGTGCCAGCAGCCGCGGTAATACGTAGGTGGCAAGCGTTATCCG

GATTTATTGGGCGTAAAGAGAGTGCAGGCGGTTTTCTAAGTVTGATGTGAAAGCCTT

CGGCTTAACCGGAGAAGTGCATCGGAAACTGGATAACTTGAGTGCAGAAGAGGGTA

GTGGAACTCCATGTGTAGCGGTGGAATGCGTAGATATATGGAAGAACACCAGTGGCG
AAGGCGGCTACCTGGTCTGCAACTGACGCTGAGACTCGAAAGCATGGGTAGCGAAC
AGGATTAGATACCCTGGTAGTCCATGCCGTAAACGATGAGTGCTAGGTGTTGGAGGGT
TTCCGCCCTTCAGTGCCGGAGCTAACGCATTAAGCACTCCGCCTGGGGAGTACGACC
GCAAGGTTGAAACTCAAAGGAATTGACGGGGGCCCGCACAAGCGGTGGAGCATGTG
GTTTAATTCGAAGCTACGCGAAGAACCTTACCAGGTCTTGACATCTTGCGCCAACCCT
AGAGATAGGGCGTTTCCTTCGGGAACGCAATGACAG (SEQ ID NO:1).

### 3. Identification and preservation of the strains

In order to clarify the taxonomic status of the MSJK0025 strain, the strain MSJK0025 was identified from cell morphology, physiological and biochemical characteristics, and molecular biology perspectives (entrusting the Institute of Microbiology, Chinese Academy of Sciences for identification). In particular, the cell morphology and measuring results of biochemical characteristics of the MSJK0025 strain were shown in table 1.

**Table 1: the cell morphology and measuring results of biochemical characteristics of the MSJK0025 strain**

| **Test projects** | **Results** | | **Test projects** | **Results** | | **Test projects** | **Results** | |
|---|---|---|---|---|---|---|---|---|
| | **F1** | **F5** | | **F1** | **F5** | | **F1** | **F5** |
| Gram staining | Positive | Positive | cell morphology | Rod shape | Rod shape | Catalase | - | - |
| Oxidase | - | - | Mannitol | | | | | |

| **Carbohydrate acid production (API 50CH)** | | | | | | | | |
|---|---|---|---|---|---|---|---|---|
| Glycerol | - | - | Mannitol | - | - | Melezitose | - | - |
| Erythritol | - | - | Sorbitol | - | - | Raffinose | + | + |
| D-arabinose | - | - | Methyl α-D-mannopyranoside | - | - | Starch | - | - |
| L-arabinose | - | - | Methyl α-D-mannopyranoside | - | - | Glycogen | - | - |
| D-ribose | + | + | N-acetylglucosamine | - | - | Xylitol | - | - |
| D-xylose | + | + | Amygdalin | - | - | Gentiobiose | - | - |
| L-xylose | - | - | Arbutin | - | - | D-turanose | - | - |
| Adonitol | - | - | Esculin | - | - | D-lyxose | - | |
| Sucrose | + | + | Salicin | - | - | D-tagatose | - | - |
| D-galactose | + | + | Cellobiose | - | - | D-Fucitol | - | - |
| D-glucose | + | + | Maltose | + | + | L-Fucitol | - | - |
| D-fructose | + | + | Lactose | + | + | D-Arabinitol | - | - |
| D-mannose | + | + | 2-keto-gluconate | + | + | L-Arabinitol | - | - |
| L-Sorbitol | - | - | Methyl β-D-xylobioside | - | - | Gluconate | - | - |
| L-rhamnose | - | - | Trehalose | - | - | Melibiose | + | + |
| Dulcitol | - | - | Inulin | - | - | Inositol | - | - |

| | | | | | | | | |
|---|---|---|---|---|---|---|---|---|
| Explanatory note: F1 in table 1 represents first generation MSJK0025 strain, and F5 in table 1 represents fifth generation MSJK0025 strain. | | | | | | | | |

From the test data in table 1 such as cell morphology, measuring results of physiological and biochemical characteristics of the strain MSJK0025, 16S rRNA gene sequence (the sequence number in GenBank is OP861710.1) of strain MSJK0025 and referencing to some related research paper such as "Bergry's Manual of Systematic Bacteriology" and "International Journal of Systematic and Evolutionary Microbiology", the strain was identified as *"Limosilactobacillus fermentum"* and officially named as *"Limosilactobacillus fermentum* MSJK0025". The strain was preserved in China General Microbiological Culture Collection Center, with a preservation number of CGMCC No.26426, and the address of the preservation unit is Institute of Microbiology, Chinese Academy of Sciences, No. 3, Yard 1, Beichen West Road, Chaoyang District, Beijing, China.

### Example 2: hemolysis test of Limosilactobacillus fermentum MSJK0025

After the *Limosilactobacillus fermentum* MSJK0025 was activated, it was inoculated on blood plate, and cultured under anaerobic condition of 36 °C for 48 h to observe its hemolysis while the hemolytic Staphylococcus was the positive control group. The plate picture after culturing the *Limosilactobacillus fermentum* MSJK0025 was shown in FIG.6.

As shown in FIG.6, the positive control group (hemolytic Staphylococcus) shows bacterial colony growth and hemolysis phenomenon. The *Limosilactobacillus fermentum* MSJK0025 shows bacterial colony growth and no hemolysis phenomenon. It is indicated that the *Limosilactobacillus fermentum* MSJK0025 has no hemolysis, which cannot cause bacterial hemolytic diseases and have good safety.

Example 3: antibiotics sensitivity test of the *Limosilactobacillus fermentum* MSJK0025 The sensitivity of the *Limosilactobacillus fermentum* MSJK0025 to 30 kinds of common antibiotics was tested by K-B method and the antibiotics sensitivity of F1 generation strain and F5 generation strain were tested, and the measuring results were shown in table 2.

**Table 2: measuring results of antibiotics sensitivity of the Limosilactobacillus fermentum MSJK0025**

| **Antibiotics** | **Sensitivity** | | **Antibiotics** | **Sensitivity** | | **Antibiotics** | **Sensitivity** | |
|---|---|---|---|---|---|---|---|---|
| | **F1** | **F5** | | **F1** | **F5** | | **F1** | **5** |
| Piperacillin/Tazobactam | S | S | Meropenem | S | S | Rifampicin | S | S |
| Spectinomycin | R | R | Vancomycin | R | R | Tetracycline | S | S |
| Ciprofloxacin | R | R | Netilmicin | S | S | Compound sulfamethoxazole | S | S |
| Penicillin | S | S | Ceftriaxone | S | S | Amikacin | R | R |
| Erythromycin | S | S | Cefaclor | S | S | Ceftazidime | S | S |
| Chloramphenicol | S | S | Cefazolin | S | S | Cefoxitin | S | S |
| Azithromycin | S | S | Cefotaxime | S | S | Cefoperazone | S | S |
| Clindamycin | S | S | Ampicillin | S | S | Gentamicin | R | R |
| Strong cycline | S | S | Cefuroxime | M | M | Mezlocillin | S | S |
| Clarithromycin | S | S | Minocycline | S | S | Furantoin | S | S |
| Explanation: S: Sensitivity; M: Moderate sensitivity; R: Resistance. | | | | | | | | |

| | | | | | | | | |
|---|---|---|---|---|---|---|---|---|
| Explanatory note: F1 in table 2 represents first generation MSJK0025 strain, and F5 in table 2 represents fifth generation MSJK0025 strain. | | | | | | | | |

As can be seen from table 2 that, the *Limosilactobacillus fermentum* MSJK0025 has good antibiotics sensitivity and no drug-resistance to most of antibiotics, and its property can be stably inherited.

### Example 4: sequencing and analysing of the complete genome sequence of the Limosilactobacillus fermentum MSJK0025

The complete genome sequence of the *Limosilactobacillus fermentum* MSJK0025 was sequenced, which was submitted to Genebank to obtain a sequence number of CP121468.1. An ANI value (Aversge nucleotide identity, ANI) between the complete genome sequence of the strain and the genome sequence of standard strain of the *Limosilactobacillus fermentum* is 97.6284%.

The analysing results of complete genome sequence data of the *Limosilactobacillus fermentum* MSJK0025 were shown in tables 3-5.

**Table 3: genes related to adjusting gut microbiota in the Limosilactobacillus fermentum MSJK0025**

| **Probiotic Determinants** | **Gene Number** | **Gene Name** |
|---|---|---|
| Antibacterial activity | 0 | - |
| Exopolysaccharide(EPS) | 5 | epsA, LGAS_RS05680, LAF _RS00460, LAF_RS00465, LAF_RS08090 |

**Table 4: genes related to survival in gut in the complete genome sequence of the Limosilactobacillus fermentum MSJK0025**

| **Survival in Gut** | **Gene Number** | **Gene Name** |
|---|---|---|
| Acid resistance | 5 | LBA1524, LBA1272, lr1516, dltD, dltA |
| Adaptation | 2 | Lr1265, Lr1584 |
| Adherence | 4 | FbpA, ispA, MapA, 32-Mmubp |
| Bile resistance | 7 | LBA1430, clpE, dps, LBA1446, LBA1680, clpL, LBA1431 |
| Competitive ability | 3 | copA, met, pts14C |
| Growth | 0 | - |
| Persistence | 2 | msrB, clpC |

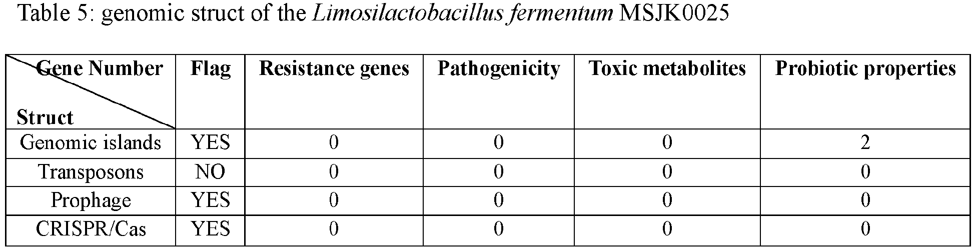

After analysing the complete genome sequence data of the *Limosilactobacillus fermentum* MSJK0025 in the tables 3-5, the obtained conclusions were as follows:
(1) the virulence factors are not detected, so the strain has no pathogenic hazards;
(2) the antibiotic resistance genes are not detected;
(3) the strain has exopolysaccharide which facilitates survival in gut; and
(4) the strain has good acid resistance, adherence, bile resistance, competitive ability and persistence, which facilitates survival in gut.

### Example 5: in vitro resistance tests of the Limosilactobacillus fermentum MSJK0025

### 1. Test schemes

The control groups in the following tests were the famous commercial strain *Limosilactobacillus fermentum* CECT5716; and test groups were the *Limosilactobacillus fermentum* MSJK0025.

Acid resistance test: MRS broth medium was basic medium. The strains were inoculated in control medium (the basic medium with pH 6.0), and basic MRS medium with pH 2.0, pH 3.0, pH 4.0 and pH 5.0, respectively, cultured at 37 °C for 17 h, diluted in 10 times series gradient by sterile saline and conducted colony counting. The survivability determination formula was:
*P*=lg*P*₁/lg*P*₀, in which P was the logarithmic ratio of live bacteria of tested strains, %; *P*₀ was the number of live bacteria of control group, CFU/mL; and *P*₁ was the number of live bacteria of test group, CFU/mL.

Bile salt resistance test: the strain liquid was cultured in MRS broth medium for 17 h. 1 mL bacterial fluid was sucked and inoculated in 4 sterile centrifuge tubes containing 10 mL of MRS broth medium. The high concentration bile salt mother liquor was prepared and filtered to remove bacteria, and added into the sterile centrifuge tubes to adjust the bile salt content to 0%, 0.1%, 0.2% and 0.3%, respectively, cultured at 36 °C, and determined the number of live bacteria at 0 h, 2 h, 4 h and 6 h. The survivability determination formula was:
*P*=lg*P*₁/lg*P*₀, in which *P* was survivability of tested strains, %; *P*₀ was the number of live bacteria of control group, CFU/mL; and *P*₁ was the number of live bacteria of test group, CFU/mL.

Artificial gastric juice and intestinal juice resistance test: the *Limosilactobacillus fermentum* MSJK 0025 and *Limosilactobacillus fermentum* CECT5716 were cultured in MRS broth medium, respectively, and the bacterial fluids were cultured for 17 h. The bacterial fluids were shaken well, and 10 mL strain liquids were taken to be centrifuged at a speed of 4000 rpm for 4 min to obtain bacterial sludges, respectively. The bacterial sludges were washed by PBS buffer solution for 3 times, and resuspended in 10 mL artificial gastric juice / artificial intestinal juice for heat preservation and digestion at 37 °C, respectively. Samples were taken at 0 h, 1 h, 2 h and 3 h, respectively, to test the number of live bacteria in artificial gastric juice group. Samples were taken at 0 h, 2 h, 4 h and 6 h, respectively, to test the number of live bacteria in artificial intestinal juice. The survivability determination formula was:
*P*=lg*P*₁/lg*P*₀, in which *P* was the logarithmic ratio of live bacteria of tested strains, %; *P*₀ was the number of live bacteria after digestion for 0 h, CFU/mL; and P₁ was the number of live bacteria after digestion for *n* h, CFU/mL.

### 2. Results and analysis

### (1) Acid resistance

Lactic acid bacteria will generate a large amount of organic acids in the metabolic process, so they have a certain of acid resistance, and this is one of the key factors for probiotics to pass through the gastric juice barrier. In addition, acid resistance is an important factor affecting the yield of the strain in the fermentation process. The survivability of the *Limosilactobacillus fermentum* MSJK0025 and CECT5716 cultured at different pH values were shown in table 6.

**Table 6: the results of acid resistance of the Limosilactobacillus fermentum MSJK0025 and CECT5716**

| **Processing methods** | **Logarithmic ratio of live bacteria of MSJK0025 (%)** | **Logarithmic ratio of live bacteria of CECT5716 (%)** |
|---|---|---|
| CK (MRS medium with pH 6.0) | 100.00 | 100.00 |
| MRS broth medium with pH 2.0 | 56.90 | 58.21 |
| MRS broth medium with pH 3.0 | 69.14 | 66.35 |
| MRS broth medium with pH 4.0 | 82.18 | 83.06 |
| MRS broth medium with pH 5.0 | 88.72 | 89.23 |

As can be seen from table 6 that, with the increase of pH value, logarithmic ratios of live bacteria of the strains MSJK0025 and CECT5716 shows an increasing trend, and the increase amplitudes of the two are quite similar.

### (2) Bile salt resistance

The bile is secreted by the liver and enters "the duodenum (a part of the small gut)" through the gallbladder, which has good killing effect to the probiotics. A concentration of the bile salt of the duodenum fluctuates from 0.03% to 0.3%. However, a retention time of the food in the duodenum is about 4-6 h. The survivability of the *Limosilactobacillus fermentum* MSJK 0025 and CECT5716 at different concentrations of the bile salt were shown in table 7.

**Table 7: the results of bile salt resistance of the Limosilactobacillus fermentum MSJK 0025 and CECT5716**

| Processin g methods | Logarithmic ratio of live bacteria after digestion for 0 h (%) | | Logarithmic ratio of live bacteria after digestion for 2 h (%) | | Logarithmic ratio of live bacteria after digestion for 4 h (%) | | Logarithmic ratio of live bacteria after digestion for 6 h (%) | |
|---|---|---|---|---|---|---|---|---|
| | MSJK00 25 | CECT57 16 | MSJK00 25 | CECT57 16 | MSJK00 25 | CECT57 16 | MSJK00 25 | CECT57 16 |
| 0.1% bile salt | 100.00 | 100.00 | 33.72 | 47.72 | 33.54 | 46.39 | 32.94 | 45.79 |
| 0.2% bile salt | 100.00 | 100.00 | 21.01 | 0 | 0 | 0 | 0 | 0 |
| 0.3% bile salt | 100.00 | 100.00 | 0 | 0 | 0 | 0 | 0 | 0 |

As can be seen from table 7 that, the bile salt resistance of strain MSJK0025 is slightly lower than that of strain CECT5716, but the difference is not significant.

### (3) Artificial gastric juice resistance

In normal circumstance, the pH of gastric juice when people are on an empty stomach is 0.9-1.5, and the pH of gastric juice after a meal is above 3.5. However, the gastric emptying time is about 3 h. The survivability of the *Limosilactobacillus fermentum* MSJK0025 and CECT5716 at different pH values were shown in table 8.

**Table 8: the results of artificial gastric juice resistance of the Limosilactobacillus fermentum MSJK0025 and CECT5716**

| Processing methods | Logarithmic ratio of live bacteria after digestion for 1 h (%) | | Logarithmic ratio of live bacteria after digestion for 2 h (%) | | Logarithmic ratio of live bacteria after digestion for 3 h (%) | |
|---|---|---|---|---|---|---|
| | MSJK0025 | CECT5716 | MSJK0025 | CECT5716 | MSJK0025 | CECT5716 |
| Artificial gastric juice with pH 1.5 | 21.20 | 0.00 | 0.00 | 0.00 | 0.00 | 0.00 |
| Artificial gastric juice with pH 2.0 | 76.55 | 76.71 | 76.44 | 69.09 | 76.44 | 63.36 |
| Artificial gastric juice with pH 2.5 | 99.89 | 100.00 | 99.68 | 97.77 | 99.79 | 97.66 |
| Artificial gastric juice with pH 3.0 | 99.79 | 99.67 | 99.79 | 96.71 | 99.58 | 96.93 |
| Artificial gastric juice with pH 3.5 | 99.79 | 99.89 | 99.79 | 97.50 | 99.79 | 97.18 |

As can be seen from table 8 that, the *Limosilactobacillus fermentum* MSJK0025 still has a survivability of 21.20% after being treated in the artificial gastric juice resistance with pH 1.5 for 1 h, but the live bacteria are not detected in the strain CECT5716. With the increase of pH, their resistances synchronously increased. Taken as a whole, the acid resistance of the *Limosilactobacillus fermentum* MSJK0025 is better than that of the strain CECT5716.

### (4) Artificial intestinal juice resistance

The gut is the main place for survive of the probiotic in the digestive tract. The survivability of the *Limosilactobacillus fermentum* MSJK0025 and CECT5716 processed in the artificial intestinal juice at different processing times were shown in table 9.

**Table 9: the results of artificial intestinal juice resistance of the Limosilactobacillus fermentum MSJK0025 and CECT5716**

| Processing methods | Logarithmic ratio of live bacteria of MSJK0025 (%) | Logarithmic ratio of live bacteria of CECT5716 (%) |
|---|---|---|
| Digestion at 37 °C for 0 h | 100.00 | 100.00 |
| Digestion at 37 °C for 2 h | 99.58 | 99.79 |
| Digestion at 37 °C for 4 h | 98.75 | 99.79 |
| Digestion at 37 °C for 6 h | 98.85 | 99.58 |

As can be seen form table 9 that, with the prolonging of digestion time, the logarithmic ratio of live bacteria of the strain MSJK0025 and CECT5716 are both slightly decreased, but the decrease amplitudes were small and the difference between them was not significant. The logarithmic ratios of live bacteria after digestion for 6 h are both around 99%. The data indicates that the strain MSJK0025 has a strong survivability in the artificial intestinal juice, which is close to that of strain CECT5716.

### 3. Conclusion

*Limosilactobacillus fermentum* CECT5716 from Biosearch Life of Spain is a famous commercial strain. The measuring results show that the difference between acid resistance, bile salt resistance, artificial gastric juice resistance, and artificial intestinal juice resistance of strain MSJK0025 and that of *Limosilactobacillus fermentum* CECT5716 is not significant.

### Example 6: Survival detection of the Limosilactobacillus fermentum MSJK0025 in the gut of zebrafish

### 1. MTC (maximum test concentration) detection of survival in gut

Wild type AB strain zebrafish 5 days after fertilization were randomly selected to be placed in 6-hole plate, and 30 zebrafish were treated in each hole (test group). The provided samples were diluted in water (their concentrations were shown in table 10), normal control group was provided, and the capacity of each role was 3 mL. After being treated at 28 °C for 24 h, MTC of survival in gut of normal zebrafish from test samples were tested, and the results were shown in table 10.

**Table 10: test results of survival in gut of the Limosilactobacillus fermentum MSJK0025 (n=30)**

| **Groups** | **Count of zebrafish (CFU/mL)** | **Number of deaths** | **Mortality rate (%)** | **Phenotype** |
|---|---|---|---|---|
| Normal control group | - | 0 | 0 | No obvious abnormal phenomenon |
| *Limosilactobacillus fermentum* MSJK0025 | 3.12 × 10⁸ | 0 | 0 | Similar with the state of normal control group |
| | 6.25 × 10⁸ | 0 | 0 | Similar with the state of normal control group |
| | 1.25 × 10⁹ | 0 | 0 | Similar with the state of normal control group |
| | 2.50 × 10⁹ | 0 | 0 | Similar with the state of normal control group |
| | 5.00 × 10⁹ | 4 | 13 | - |

As can be seen form table 10 that, MTC of survival in gut of the *Limosilactobacillus fermentum* MSJK0025 is 2.50×10⁹ CFU/mL.

### Test the time of survival in gut

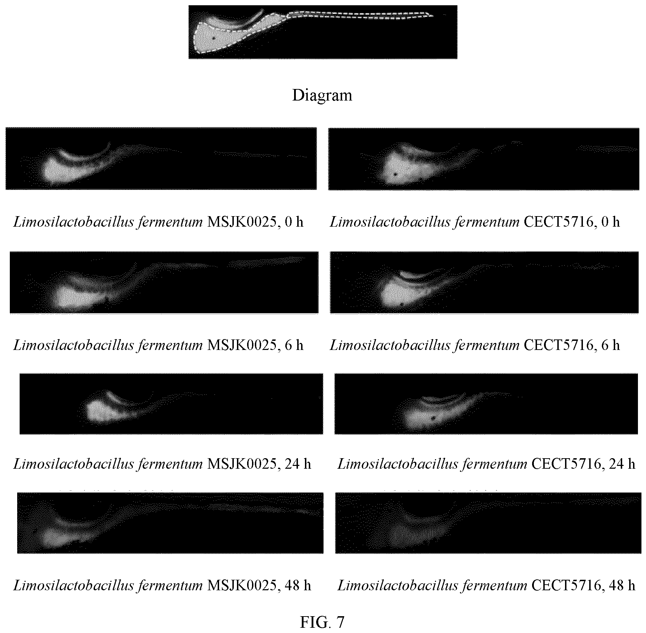

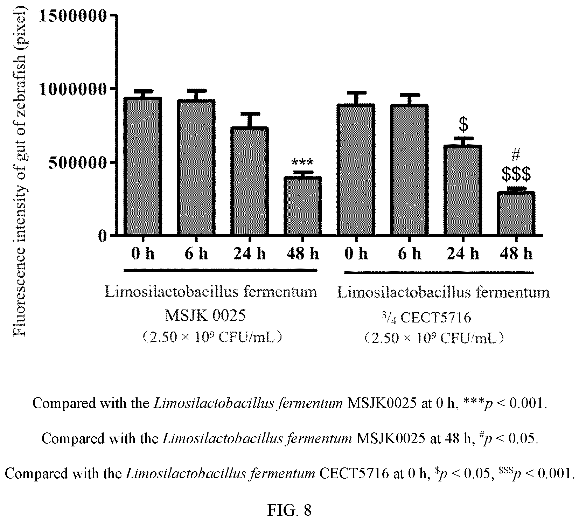

The *Limosilactobacillus fermentum* MSJK0025 and the Lactobacillus fermentum CECT5716 were marked by DIO green fluorescence. Wild type AB strain zebrafish 5 days after fertilization were randomly selected to be placed in 6-hole plate, and 30 zebrafish were treated in each hole (test groups). 2.50×10⁹ CFU/mL of the *Limosilactobacillus fermentum* MSJK0025 and 2.50×10⁹ CFU/mL of the Lactobacillus fermentum CECT5716 were diluted in water, and the capacity of each role was 3 mL. Four biological replications were parallelly provided. After being treated at 28 °C for 24 h, the probiotic was removed. After continuously being treated for 0, 6, 24 and 48 h, 10 zebrafish in each test group were randomly selected and placed under a fluorescence microscope to be observed and pictured, and the picture was preserved (shown in FIG. 7). The data were analyzed and collected by NIS-Elements D 3.20 advanced image processing software, and fluorescence intensity of gut of zebrafish was analyzed, then the survival in gut was evaluated according to statistical analysis results of corresponding indices, and the results were shown in table 11 and FIG. 8. The statistical analysis results were represented by mean ± SE. The statistical analysis was conducted by SPSS 26.0 software, and if *p* was less than 0.05, the difference had statistical significance.

**Table 11: survival test results of the Limosilactobacillus fermentum MSJK0025 in gut of zebrafish (n=10)**

| **Groups** | **Time after removing probiotic (h)** | **Fluorescence intensity of gut of zebrafish (pixel, mean ± SE)** |
|---|---|---|
| *Limosilactobacillus fermentum* MSJK0025 (2.50 × 10⁹ CFU/mL) | 0 | 933626 ± 46990 |
| | 6 | 916866 ± 67467 |
| | 24 | 731236 ± 96801 |
| | 48 | 393525 ± 36981*** |
| *Limosilactobacillus fermentum* CECT5716 (2.50 × 10⁹ CFU/mL) | 0 | 886263 ± 86050 |
| | 6 | 884237 ± 72234 |
| | 24 | 607969 ± 54335^{$} |
| | 48 | 289585 ± 30089^{#$$$} |

Explanatory note: compared with the *Limosilactobacillus fermentum* MSJK0025 at 0 h, ****p* was less than 0.001; compared with the *Limosilactobacillus fermentum* MSJK0025 at 48 h, ^{#}*p* was less than 0.05; and compared with the *Limosilactobacillus fermentum* CECT5716 at 0 h, ^{$}*p* was less than 0.05 and ^{$$$}*p* was less than 0.001.

As can be seen from table 11 and FIG. 8 that, under the test conditions, the *Limosilactobacillus fermentum* MSJK0025 and the *Limosilactobacillus fermentum* CECT5716 both can survive in the gut of zebrafish, and their effective survival times are 24 h and 6 h, respectively. The fluorescence intensity of two *Limosilactobacillus fermentum* in the gut of zebrafish at 0, 6 and 24 h have no difference, but they have obvious difference at 48 h.

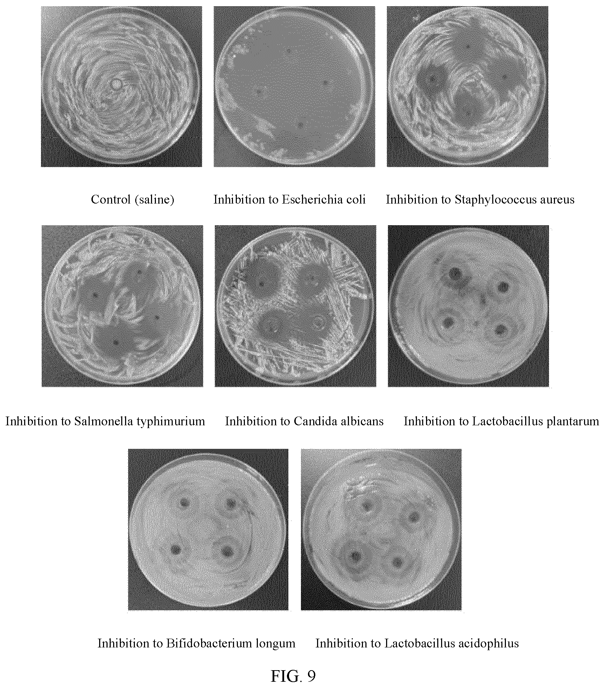

Example 7: bacteriostasis experiment of the *Limosilactobacillus fermentum* MSJK0025 The *Limosilactobacillus fermentum* MSJK0025 was cultured in MRS liquid culture medium for 17 h. Escherichia coli, Staphylococcus aureus, Salmonella typhimurium, Candida albicans, Lactobacillus plantarum, Bifidobacterium longum and Lactobacillus acidophilus were indicator bacteria, which were activated to prepare bacterial fluid. The bacterial fluid was uniformly coated on nutrient agar plate. An oxford cup was vertically placed on the surface of the plate, and pressed to tightly contact the culture medium. 200 µL *Limosilactobacillus fermentum* MSJK0025 bacterial fluid was sucked into the oxford cup, and cultured at 37 °C for 24 h. The diameters of inhibition zone were measured, the results were shown in table 12 and FIG.9.

**Table 12: antibacterial test results of the Limosilactobacillus fermentum MSJK0025**

| **Indicator bacteria** | **Average diameter of inhibition zone (mm)** |
|---|---|
| Escherichia coli | 30.92 |
| Staphylococcus aureus | 16.22 |
| Salmonella typhimurium | 12.64 |
| Candida albicans | 11.42 |
| Lactobacillus plantarum | - |
| Bifidobacterium longum | - |
| Lactobacillus acidophilus | - |

As can be seen form table 12 that, the *Limosilactobacillus fermentum* MSJK0025 has an inhibition effect to the pathogenic bacteria in the gut such as Escherichia coli, Staphylococcus aureus, Salmonella typhimurium, and Candida albicans, and the ranking of their inhibition effects is that: Escherichia coli > Staphylococcus aureus > Salmonella typhimurium > Candida albicans. However, *Limosilactobacillus fermentum* MSJK 0025 has no inhibition effect to the probiotic in the gut such as Lactobacillus plantarum, Bifidobacterium longum and Lactobacillus acidophilus.

### Example 8: adjusting effect of the Limosilactobacillus fermentum MSJK 0025 to the gut microbiota

30 healthy mice were selected and randomly divided into two groups, one was test group (15 mice), these mice were fed with 1.5 mL culture solution of *Limosilactobacillus fermentum* MSJK 0025 every day for five consecutive days, the number of live bacteria in the culture solution of *Limosilactobacillus fermentum* MSJK 0025 was greater than 1 × 10⁹ CFU/mL; the other was control group, these mice were fed with 1.5 mL blank MRS culture medium for five consecutive days. Feces of mice was detected to obtain microbial changes in gut of mice, the measuring results were represented by lg10ⁿ/mL, and the results were shown in table 13. In particular, the statistical analysis results were represented by mean ± SE. The statistical analysis was conducted by SPSS 26.0 software, and if *p* was less than 0.05, the difference had statistical significance.

**Table 13: adjusting effect of the Limosilactobacillus fermentum MSJK 0025 to the gut microbiota**

| **Gut bacteria** | **Control groups** | **Test groups** |
|---|---|---|
| Lactobacillus | 8.47 ± 0.53 | 9.38 ± 0.48** |
| Bifidobacterium | 9.26 ± 0.32 | 10.21 ± 0.37** |
| Escherichia coli | 7.48 ± 0.36 | 7.41 ± 0.29 |
| Clostridium perfringens | 6.14 ± 0.41 | 6.01 ± 0.52 |

Explanatory note: compared with control group, ***p* was less than 0.05.

As can be seen from table 13 that, compared with control group, after the mice are fed with the *Limosilactobacillus fermentum* MSJK0025, the contents of lactobacillus and Bifidobacterium in gut of mice are obviously increased, but the contents of Escherichia coli and Clostridium perfringens are decreased to a certain extent. It is indicated that the *Limosilactobacillus fermentum* MSJK0025 have adjusting effect to the gut microbiota.

It should be noted that, the above embodiments are only used for explaining the present application and do not limit the present application. The present application has been described with reference to typical examples, but it should be understood that the words used therein are descriptive and explanatory, not restrictive. The present application can be modified within the scope of the claims of the present application as stipulated, and changed within the scope and spirit of the present application. Although the described present application involves specific methods, materials, and examples, but this does not mean that the present application is limited to the disclosed specific examples. On the contrary, the present application can be extended to all other methods and applications with the same functionality.

## Claims

1. A *Limosilactobacillus fermentum* MSJK0025 from breast milk for adjusting gut microbiota, **characterized in that**, the *Limosilactobacillus fermentum* MSJK0025 from breast milk is preserved in China General Microbiological Culture Collection Center, with a preservation number of CGMCC No.26426.

2. The *Limosilactobacillus fermentum* from breast milk according to claim 1, **characterized in that**, 16S rRNA nucleotide sequence of the *Limosilactobacillus fermentum* MSJK0025 from breast milk is shown in SEQ ID NO:1, with a login number of OP861710.1 in GeneBank.

3. The *Limosilactobacillus fermentum* from breast milk according to claim 1 or 2, **characterized in that**, a login number of a complete genome sequence of the *Limosilactobacillus fermentum* MSJK0025 from breast milk is CP121468.1 in GeneBank.

4. An application of the *Limosilactobacillus fermentum* MSJK0025 from breast milk according to any one of claims 1-3 in preparing a probiotic composition for inhibiting pathogenic bacteria in the gut.

5. The application according to claim 4, **characterized in that**, the pathogenic bacteria in the gut comprises one or more selected from the group consisting of Escherichia coli, Staphylococcus aureus, Salmonella typhimurium, and Candida albicans.

6. The application according to claim 4, **characterized in that**, the probiotic composition is an oral formulation.

7. A probiotic composition, **characterized in that**, the probiotic composition comprises the *Limosilactobacillus fermentum* from breast milk according to any one of claims 1-3.

8. A medicine for adjusting balance of gut microbiota, **characterized in that**, the medicine comprises the *Limosilactobacillus fermentum* from breast milk according to any one of claims 1-3.

9. A food for adjusting balance of gut microbiota, **characterized in that**, the food comprises the *Limosilactobacillus fermentum* from breast milk according to any one of claims 1-3.
